# EUROPEAN PATENT SPECIFICATION

(11) **EP 0 531 461 B1**
(45) Date of publication and mention of the grant of the patent: **23.11.1994**
(21) Application number: 91920995.7
(22) Date of filing: 17.04.1991
(51) Int. Cl.: C07K 7/20, C07K 7/26, C07K 7/06, A61K 37/24, A61K 37/43

(54) **BIOLOGICALLY ACTIVE PEPTIDES CONTAINING D-2-ALKYLTRYPTOPHAN**
D-2-ALKYLTRYPTOPHAN ENTHALTENDE BIOLOGISCH AKTIVE PEPTIDE
PEPTIDES BIOLOGIQUEMENT ACTIFS CONTENANT DU D-2-ALKYLTRYPTOPHANE

(30) Priority: 11.05.1990 IT 2027390
(43) Date of publication of application: 17.03.1993
(73) Proprietor: DEGHENGHI, Romano, CH-1264 S.-Cergue (CH)
(72) Inventor: DEGHENGHI, Romano, CH-1264 S.-Cergue (CH)
(74) Representative: Minoja, Fabrizio
(86) International application number: EP9100727
(87) International publication number: WO9118016

(56) References cited:
- EP-A- 83 864
- EP-A- 203 031
- US-A- 3 316 260
- NEW ENGLAND JOURNAL OF MEDICINE, 29 March 1990; R.M. SILVER et al., pp. 874-881#
- CHEM. PHARM. BULLETIN, vol. 27, no. 8, 1979; Y. YABE et al., pp.1907-1911#
- ENDOCRINE REVIEWS, vol. 7, no. 1, 1986, Endocrine Society (US); M.J. KARTEN et al., pp. 44-66#
- PROCEEDINGS OF THE NATL. ACADEMY OF SCIENCES USA, vol. 83, 1986; Y. TORRENS et al., pp. 9216-9220#

## Description

### Technical Field

The present invention relates to the field of biologically active peptides. Specifically, this invention relates to biologically active peptides containing the amino acid D-Tryptophan (D-Trp).

### Background Art

It is well known that the incorporation or substitution of a D-Tryptophan residue into a biologically active peptide chain enhances the activity of that chain. Furthermore, such incorporation or substitution will prolong the biological activity. The prolonged and enhanced effectiveness of such peptides probably relates the increased resistance to degradation by peptidases.

Examples of D-Tryptophan containing peptides are the LHRH agonists as described by D.H. Coy et al., Journal of Medical Chemistry, volume 19, page 423 (1976), W. Koenig et al., Peptide Chemistry (1987), T. Shiba and S. Sakakibara (eds.), Osaka, Protein Research Foundation, Osaka (1988), page 591, B.J.A. Furr et al., Journal of Endocrinol. Invest., volume 11, page 535 (1988). Examples of D-Tryptophan containing somastostatin analogs, such as the peptides octreotide and vapreotide are disclosed by R. Deghenghi, Biomedicine and Pharmacotherapy, volume 42, page 585 (1988). Another example of a D-Tryptophan containing peptide are the synthetic antagonists of Substance P as disclosed by D. Regoli et al., European Journal of Pharmacology, volume 99, page 193, (1984), and GHRP-6 described by C.Y. Bowers et al., Endocrinology, volume 114, page 1537, (1984).

Peptides containing Tryptophan have been subject to degradation due to the "Kynurenine pathway". In this pathway, the enzyme Tryptophan pyrrolase (i.e., indolamine 2,3-dioxygenase) degrades the pyrrole ring of Tryptophan. Kynurenine and other breakdown products are generated by this degradation. Some of the breakdown products have been shown to be toxic when present in elevated concentrations as reported by R.M. Silver et al., The New England Journal of Medicine, volume 322, page 874, (1990).

D-Tryptophan containing peptides are subject to degradation by oxygen and other reactive radicals as reported by R. Geiger and W. Koenig, "The Peptides," Academic Press, volume 3, page 82, New York (1981). The D-Tryptophan in the peptide chain may react with active or activated groups when peptides are formulated in certain controlled delivery pharmaceutical compositions, such as those based on polylactic-polyglycolic acid polymers. Such degradation is thought to be facilitated by either heat or by the presence of catalysts. It is also possible that radiolysis products formed during ionizing sterilization of these pharmaceutical compositions may facilitate the breakdown of D-Tryptophan. Clearly, the breakdown of D-Tryptophan, and the concomitant breakdown of the pharmaceutical compound containing D-Tryptophan is an undesirable effect.

What is needed is a derivative of D-Tryptophan which retains the prolonged and increased biological activity discussed above, while resisting degradation by indolamine dioxygenase, oxygen, or other reactive radicals. It is of course essential that such a derivative of D-Tryptophan would maintain biological activity as compared to natural D-Tryptophan containing bioactive peptides.

The terms "biological effect" or "pharmacological effect" as used in the present disclosure refer to the qualitative effect that a bioactive peptide has upon living tissue. As an example, LHRH, luteinizing hormone releasing hormone, has the biological effect of causing cells of the anterior pituitary gland to release luteinizing hormone. In contrast, the term "potency" is used in its conventional sense to refer to the degree and duration of the bioactivity of a given peptide.

Utilizing these terms as defined above, what is needed is a Tryptophan containing bioactive peptide which is resistant to oxidative degradation and reactive radical attack while maintaining the same biological activity and a similar or greater potency than the presently available analogous peptides provide.

### Summary of Invention

Now in accordance with the present invention, a derivative of D-Tryptophan has been discovered which imparts to a biologically active peptide incorporating the derivative improved resistance to oxidative breakdown reactions of the Tryptophan derivative, while maintaining the biological activity and pharmacological effect exhibited by peptides incorporating unaltered D-Tryptophan. Specifically, the present invention relates to biologically active peptides incorporating at least one D-Tryptophan in which a lower alkyl group, preferably an alkyl group containing 1 to 3 carbons, is substituted in the 2 position. Peptides incorporating such substituted D-Tryptophans are more stable in the presence of reactive radicals, or when pharmaceuticals containing such peptides are exposed to ionizing radiation.

### Detailed Description

Biologically active peptides in accordance with the present invention are characterized by the following formula:

A-D-2-ALK-TRP-B

where alk is a lower alkyl group, preferably comprising 1 to 3 carbons. A is SEQUENCE ID NO:1, wherein the Glutamic Acid residue at SEQUENCE position 1 is Pyro-Glu; D-Phe-Cys-Phe; SEQUENCE ID NO:2 wherein the Phenylalanine residue at SEQUENCE position 1 is D-Phe, the Proline residue at SEQUENCE position 4 is D-Proline and the Tryptophan residues at SEQUENCE positions 7 and 9 are D-Tryptophan; Arg-D-Trp-N-methyl-Phe; or His; and B is SEQUENCE ID NO:3 wherein the Glycine residue at sequence position 4 is Gly-NH₂; Leu-Arg-Pro-NHCH₂CH₃; Lys-Thr-Cys-NHCH(CH₂OH)CHOH(CH₃); SEQUENCE ID NO:4 wherein the Tryptophan residue at SEQUENCE position 4 is Trp-NH₂; Met-NH₂; Leu-Met-NH₂; SEQUENCE ID NO:5 wherein the Phenylalanine residue at SEQUENCE position 3 is D-Phe and the Lysine residue at SEQUENCE position 4 is Lys-NH₂; provided that when A is SEQUENCE ID NO:1, B is SEQUENCE ID NO:3 wherein the Glycine residue at SEQUENCE position 4 is Gly-NH₂ or Leu-Arg-Pro-NHCH₂CH₃; when A is D-Phe-Cys-Phe, B is Lys-Thr-Cys-NHCH-(CH₂OH)CHOH(CH₃) wherein the cysteine units are bound to each other by a cyclic disulphide; when A is D-Phe-Cys-Tyr, B is SEQUENCE ID NO:4 wherein the cysteine units are bound to each other by a cyclic disulfide and the Tryptophan residue at SEQUENCE position 4 is Trp-NH₂; when A is SEQUENCE ID NO:6 wherein the Proline residue in SEQUENCE position 1 is D-Pro and the Tryptophan residues at SEQUENCE positions 4 and 6 are D-Trp, B is Met-NH₂; when A is Arg-D-Trp-n-methyl-Phe, B is Leu-Met-NH₂; or when A is His, B is SEQUENCE ID NO:5 wherein the Phenylalanine residue at SEQUENCE position 3 is D-Phe and the Lysine residue at SEQUENCE position 4 is Lys-NH₂.

The following compounds are biologically active peptides of the present invention which contain at least one C-2 substituted D-Tryptophan according to the present invention.
A. SEQUENCE ID NO:7 wherein the Glutamic acid residue at SEQUENCE position 1 is Pyro-Glu, the Tryptophan residue at SEQUENCE position 6 is D-2-methyl-Trp, and the Glycine residue at SEQUENCE position 10 is Pro-Gly-NH₂.
B. SEQUENCE ID NO:8 wherein the Glutamic Acid residue at SEQUENCE position 1 is Pyro-Glu, the Tryptophan residue at SEQUENCE position 6 is D-2-methyl-Trp, and the Proline residue at SEQUENCE position 9 is Pro-NHCH₂CH₃.
C. SEQUENCE ID NO:9 wherein the Proline residue at SEQUENCE position 1 is D-Pro, the Tryptophan residue at SEQUENCE position 4 is D-Trp, the Tryptophan residue at SEQUENCE position 6 is D-Trp, the Tryptophan residue at SEQUENCE position 7 is D-2-ethyl-Trp, and the Methionine residue at SEQUENCE position 8 is Met-NH₂.
D. SEQUENCE ID NO:10 wherein the Tryptophan residue at SEQUENCE position 2 is D-Trp, the Phenylalanine residue at SEQUENCE position 3 is N-methyl-Phe, the Tryptophan residue at SEQUENCE position 4 is D-2-isopropyl-Trp, and the Methionine residue at SEQUENCE position 6 is Met-NH₂.
E. SEQUENCE NO:11 (and its cyclic oxidation product) wherein the Phenylalanine residue at SEQUENCE position 1 is D-Phe, the Tryptophan residue at SEQUENCE position 4 is D-2-methyl-Trp and the Cysteine residue at SEQUENCE position 7 is Cys-NHCH(CH₂OH)CHOHCH₃.
F. SEQUENCE ID NO:12 (and its cyclic oxidation product) wherein the Phenylalanine residue at SEQUENCE position 1 is D-Phe, the Tryptophan residue at SEQUENCE position 4 is D-2-methyl-Trp, and the Tryptophan residue at SEQUENCE position 8 is Trp-NH₂.
G. SEQUENCE ID NO:13 wherein the Tryptophan residue at SEQUENCE position 2 is D-2-methyl Trp, the Phenylalanine residue at SEQUENCE position 5 is D-Phe, and the Lysine residue at position 6 is Lys-NH₂.

Compounds A and B are analogs of the natural peptide SEQUENCE ID NO:14 wherein the Glutamic acid residue at SEQUENCE position 1 is Pyro-Glu and the Glycine residue at position 10 is Gly-NH₂. SEQUENCE ID NO:14 is luteinizing hormone releasing hormone (LH-RH), a neurohumoral hormone produced in the hypothalamus which stimulates the secretion of the LH luteinizing hormone by the anterior pituitary gland. Compounds A and B pertain therefore to the class of LHRH agonists and are also defined respectively as follows: [D-2-methyl-Trp⁶]LHRH and [Des-Gly¹⁰-D-2-methyl-Trp⁶-Pro-ethylamide⁹]LHRH.

Compounds C and D are antagonists of substance P. Substance P is a neurotransmitter used by sensory neurons that convey responses of pain or other noxious stimuli to the central nervous system. Compounds C and D therefore have analgesic and anti-inflammatory effects. Peptides E and F are analogs (agonists) of somatostatin and as such show antisecretory and antitumoral activity. Peptide G is an analog of GHRP (Growth Hormone Releasing Peptide) stimulating the release of growth hormone.

2-methyl-Tryptophan is known (cf. H.N. Rydon, J. Chem. Soc. 1948, 705) and the homologous alkylated derivatives are conveniently prepared from the corresponding 2-alkyl indoles by well known methods (cf. J.P. Li et al., Synthesis (1), 73, 1988). The resolution of the racemic Tryptophan derivatives to give the D-enantiomers of the present invention can also be achieved by a variety of methods (cf. Amino Acids, Peptides and Proteins, Vol. 16, pages 18-20, The Royal Society of Chemistry, London, 1985). Both the solution phase or the solid phase method of peptide synthesis can be used to make the peptides of this invention, (cf. R. Geiger et al., "The Peptides", Academic Press, New York 1981). If the solid phase method is used, peptide synthesizers such as the Applied Biosystem 430A, Bioresearch Sam 9500 or the Beckman Model 990 are preferably used.

According to this methodology, the first amino acid is linked to the benzhydrylamine resin and the remaining protected amino acids are then coupled in a step wise manner using the standard procedures recommended by the manufacturers of the synthesizers. For instance, amino acid couplings are performed by using symmetrical anhydrides in the Applied Biosystems Synthesizer and diisopropylcarbodiimide in the Bioresearch or Beckman machines. The amino acid derivatives are protected by the tertiary butoxy-carbonyl groups on the alpha-amino function during the synthesis. The functional groups present in the amino-acid in the side chain are previously protected, e.g. by acetyl(Ac), benzoyl (Bz), t-butyloxycarbonyl (Boc), benzyloxymethyl (Bom), benzyl (Bzl), benzyloxycarbonyl (Z), formyl (For), p-nitro-phenyl ester (0Np), tosyl (Tos), etc. For instance, the functional groups of Histidine are protected by benzyloxymethyl (His(Bom)), tosyl (His(Tos)), the functional groups of Tryptophan by formyl (Trp(For)), those of Serine by benzyl (Ser(Bzl)), those of Tyrosine by 2-Br-benzyloxycarbonyl (Tyr(2-Br-Z)), those of Arginine by tosyl (Arg(Tos)), those of Leucine by O-benzyl-p-tosyl (Leu(O-Bzl-p-Tos)), those of Proline by O-benzyl HCl (Pro(O-Bzl HCl)), those of Glycine by O-benzyl HCl (Gly (O-Bzl HCl)), those of Cysteine by 4-methyl-benzyl (Cys(4-Me-Bzl)), those of Lysine by benzyloxycarbonyl (Lys(Z)), those of Threonine by benzyl-OH (Thr(Bzl-OH)), those of Valine by O-benzyl-tosyl (Val(O-Bzl-p-Tos)), those of Glutamic Acid by O-benzyl (Glu(O-Bzl)), those of Methionine by P-nitrophenyl ester (Me(Onp)), and those of Alanine by O-benzyl HCl (Ala(O-Bzl HCl).

The Boc protective groups on the alpha-aminic function are removed at each stage by treatment with 60% trifluoroacetic acid ("TFA") in dichloromethane. Cleavage of Trp and Met containing peptides from the resin with simultaneous removal of all side-chain protecting groups is achieved as described by J.P. Tam et al., J. Am. Chem. Soc., Vol 105, page 6442 (1983). The crude peptides after HF cleavage are purified on a Sephadex® G-50 F column in 50% acetic acid or by preparative reverse phase HPLC using gradients of acetonitrile and water containing 0.1% trifluoroacetic acid.

The examples that follow are given for illustrative purposes only, but are not limitative of the present invention.

### Example 1

SEQUENCE ID NO:7 wherein the Glutamic acid residue at SEQUENCE position 1 is Pyro-Glu, the Tryptophan residue at SEQUENCE position 6 is D-2-methyl-Trp, and the Glycine residue at SEQUENCE position 10 is Gly-NH₂.

The protective groups for the side chains are Tosyl (Tos) for Arginine and Histidine and Bromo-benzyloxycarbonyl (2-Br-Z) for Tyrosine. The benzhydrylamine resin (2.2g) (Bachem^{R}), was cross-linked at 1% with Proline and the apparatus used was a Beckman Model 990. The amino acids protected by Boc (tert-butyloxycarbonyl) are coupled with dicyclohexylcarbodiimide. The Boc groups are removed by trifluoroacetic acid in methylene chloride.

The synthesis yielded 4.07 g of the decapeptide-resin (98% of theoretical weight gain). Part of this resin (1.5 g) was stirred at 0° centigrade for 30 minutes with HF (24 ml) and anisole (8 ml). HF was then removed as rapidly as possible (ca. 60 min) in vacuo and EtOAc was added to the thus obtained residue. Solid material was filtered, washed with EtOAc, dried, and extracted with 2 M AcOH. Lyophilization gave a white powder which was purified by gel filtration on a column (2.5 X 95 cm) of Sephadex® G-25 (fine) by elution with 2 M AcOH. The eluate portion corresponding to the major peak was then dried and eluted further on a column (2.5 X 95 cm) of Sephadex® G-25 (fine) previously equilibrated with the lower phase followed by the upper phase of the following biphasic solvent mixture n-BuOH-AcOH-H₂O (4:1:5). Elution with the upper phase gave a major peak and the peptide from this area was collected, concentrated to dryness, and lyophilized from dilute AcOH to give the titled peptide is a white powder. Amino acid analysis was consistent with the desired structure.

### Example 2

SEQUENCE ID NO:8 wherein the Glutamic Acid residue at SEQUENCE position 1 is Pyro-Glu, the Tryptophan residue at SEQUENCE position 6 is D-2-methyl-Trp, and the Proline residue at SEQUENCE position 9 is Pro-NHCH₂CH₃.

The peptide was assembled on a 1% cross-linked Pro-Merrifield resin (2.0 g, 1.0 mmol of Pro) using the same conditions and protecting groups employed in Example 1, with the exception that dinitrophenol group protection was used for the imidazole group of histidine. The peptide-resin obtained (3.45 g) was stirred with ethylamine (20 ml, 0°C) for 6 hours and excess amine was removed in vacuo. The protected peptide resin was extracted with MeOH and precipitated by the addition of a large excess of EtOAc to give 1.36 g of material. The obtained product was treated and deprotected with HF-anisole and crude peptide obtained after this treatment was purified by gel filtration followed by partition chromatography to yield the homogeneous peptide cited. Amino acid analysis was consistent with the desired structure.

### Examples 3-7

Using the above described methods with appropriate modifications well known to the skilled in the art, the following peptides are synthesized:
SEQUENCE ID NO:9 wherein the Proline residue at SEQUENCE position 1 is D-Proline, the Tryptophan residues at SEQUENCE positions 4 and 6 are D-Trp, the Tryptophan residue at SEQUENCE position 7 is D-2-ethyl-Trp, and the Methionine residue at SEQUENCE position 8 is Met-NH₂.

SEQUENCE ID NO:10 wherein the Tryptophan residue at SEQUENCE position 2 is D-Trp, the Phenylalanine residue at SEQUENCE position 3 is N-methyl-Phe, the Tryptophan residue at SEQUENCE position 4 is D-2-isopropyl-Trp, and the Methionine residue at SEQUENCE position 6 is Met-NH₂.

SEQUENCE ID NO:11 wherein the Phenylalanine residue at SEQUENCE position 1 is D-Phe, the Tryptophan residue at SEQUENCE position 4 is D-2-methyl-Trp, and the Cysteine residue at SEQUENCE position 7 is Cys-NHCH(CH₂OH)CHOHCH₃ (cyclic disulphide).

SEQUENCE ID NO:12 wherein the Phenylalanine residue at SEQUENCE position 1 is D-Phe, the Tryptophan residue at SEQUENCE position 4 is D-2-methyl-Trp, and the Tryptophan residue at SEQUENCE position 8 is Trp-NH₂ (cyclic disulphide).

SEQUENCE ID NO:13 wherein the Tryptophan residue at SEQUENCE position 2 is D-2-methyl-Trp, the Phenylalanine residue at SEQUENCE position 5 is D-Phe, and the Lysine residue at SEQUENCE position 6 is Lys-NH₂.

Although the aforementioned examples of the present invention disclose specific embodiments thereof, it is believed that the substitution of an D-2-alkylTryptophan in bioactive peptide containing at least one Tryptophan residue will yield bioactive peptides providing the advantages and benefits discussed above.

The incorporation of a D-2-alkylTryptophan in bioactive peptides as described above provides a method for prolonging and preserving the activity of such peptides. When analogous bioactive peptides not substituted with an D-2-alkylTryptophan are exposed to various processing conditions and substances, the activity of such peptides may be adversely effected. Sterilizing procedures used in the pharmaceutical industry may expose bioactive compounds to ionizing radiation. Such radiation may effect the formation of reactive radicals. Tryptophan containing peptides are particularly susceptible to attack by such radicals and such attack may render the peptide ineffective, or possibly toxic. Furthermore, various formulating compounds, such as polylactic-polyglycolic acid polymers may contain active, or activated groups which may also attack Tryptophan containing bioactive peptides. The present invention provides a method for protecting a tryptophan containing bioactive peptide from these manufacturing hazards while also increasing the peptides resistance to oxidative degradation after formulation is complete. It is believed that the presence of the alkyl group at the number 2 position of the Tryptophan increases the stability of the pyrrole ring wherein attack by reactive radicals and active or activated groups occurs.

While it is apparent that the invention herein disclosed is well calculated to fulfill the objects above stated, it will be appreciated that numerous embodiments and modification may be devised by those skilled in the art.

SEQUENCE LISTING

(1) INFORMATION FOR SEQ ID NO:1

(i) SEQUENCE CHARACTERISTICS
(A) LENGTH: 5
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:1

Glu His Trp Ser Tyr
1               5

(2) INFORMATION FOR SEQ ID NO:2

(i) SEQUENCE CHARACTERISTICS
(A) LENGTH: 9
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:2

Phe Cys Tyr Pro Gln Gln Trp Phe Trp
1               5

(3) INFORMATION FOR SEQ ID NO:3

(i) SEQUENCE CHARACTERISTICS
(A) LENGTH: 4
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:3

Leu Arg Pro Gly
1

(4) INFORMATION FOR SEQ ID NO:4

(i) SEQUENCE CHARACTERISTICS
(A) LENGTH: 4
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:4

Lys Val Cys Trp
1

(5) INFORMATION FOR SEQ ID NO:5
(i) SEQUENCE CHARACTERISTICS
(A) LENGTH: 4
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:5

Ala Trp Phe Lys
1

(6) INFORMATION FOR SEQ ID NO: 6

(i) SEQUENCE CHARACTERISTICS
(A) LENGTH: 6
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO: 6

Pro Gln Gln Trp Phe Trp
1               5

(7) INFORMATION FOR SEQ ID NO: 7

(i) SEQUENCE CHARACTERISTICS
(A) LENGTH: 10
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:7

Glu His Trp Ser Tyr Trp Leu Arg Pro Gly
1               5                   10

(8) INFORMATION FOR SEQ ID NO: 8

(i) SEQUENCE CHARACTERISTICS
(A) LENGTH: 9
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:8

Glu His Trp Ser Tyr Trp Leu Arg Pro
1               5

( 9 ) INFORMATION FOR SEQ ID NO: 9

(i) SEQUENCE CHARACTERISTICS
(A) LENGTH: 8
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:9

Pro Gln Gln Trp Phe Trp Trp Met
1               5

(10) INFORMATION FOR SEQ ID NO:10

(i) SEQUENCE CHARACTERISTICS
(A) LENGTH: 6
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:10

Arg Trp Phe Trp Leu Met
1               5

(11) INFORMATION FOR SEQ ID NO:11

(i) SEQUENCE CHARACTERISTICS
(A) LENGTH: 7
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO:11

Phe Cys Phe Trp Lys Thr Cys
1               5

(12) INFORMATION FOR SEQ ID NO: 12

(i) SEQUENCE CHARACTERISTICS
(A) LENGTH: 8
(B) TYPE: amino acid
(C) STRANDEDNESS: single
(D) TYPOLOGY: linear, and cyclical
oxidation product

(ii) MOLECULE TYPE: Peptide

(iii) HYPOTHETICAL: Yes

(xi) SEQUENCE DESCRIPTION: SEQ ID NO:12

Phe Cys Tyr Trp Lys Val Cys Trp
1               5

(13) INFORMATION FOR SEQ ID NO: 13

(i) SEQUENCE CHARACTERISTICS
(A) LENGTH: 6
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO: 13

His Trp Ala Trp Phe Lys
1               5

(14) INFORMATION FOR SEQ ID NO: 14

(i) SEQUENCE CHARACTERISTICS
(A) LENGTH: 10
(B) TYPE: amino acid
(C) STRANDEDNESS: single
(D) TYPOLOGY: linear

(ii) MOLECULE TYPE: Peptide

(iii) HYPOTHETICAL: Yes

(xi) SEQUENCE DESCRIPTION: SEQ ID NO: 14

Glu His Trp Ser Tyr Gly Leu Arg Pro Gly
1               5                   10

## Claims (Claims for the following Contracting State(s): AT, BE, CH, DE, DK, FR, GB, IT, LI, LU, NL, SE)

1. Bioactive peptides containing D-Tryptophan residues wherein at least one D-Tryptophan residue is replaced with D-2-alkylTryptophan having a lower alkyl group substituted at the number 2 position, said D-2-alkylTryptophan providing increased oxidation resistance to the peptide while maintaining substantially the same pharmacological effect as analogous bioactive peptides in which the at least one Tryptophan residue is not replaced.

2. The bioactive peptides of claim 1 wherein the lower alkyl group includes 1 to 3 carbons.

3. The bioactive peptides of claim 2 wherein the lower alkyl group is methyl, ethyl, or isopropyl.

4. Bioactive peptides characterized by the formula A-D-2-alk-Trp-B, wherein alk is a lower alkyl group;
A is 
Glu His Trp Ser Tyr
1               5
 wherein the Glutamic acid residue is pyroGlu; D-Phe-Cys-Phe; D-Phe-Cys-Tyr; 
Pro Gln Gln Trp Phe Trp
1               5
 wherein the Proline residue is D-Pro and the Tryptophan residues at positions 4 and 6 are D-Trp; Arg-D-Trp-N-methyl-Phe; or His; and
B is 
Leu Arg Pro Gly
1
 wherein the Glycine residue is Gly-NH₂; Leu-Arg-Pro-NHCH₂CH₃; Lys-Thr-Cys-NHCH(CH₂OH)CHOH(CH₃), 
Lys   Val   Cys   Trp
1
 wherein the Tryptophan residue is Trp-NH₂; Met-NH₂; Leu-Met-NH₂ or 
Ala   Trp   Phe   Lys
1
 wherein the Phenylalanine residue is D-Phe and the Lysine residue is Lys-NH₂ provided that:
when A is 
Glu His Trp Ser Tyr
 1                5
 wherein the Glutamic acid residue is pyroGlu, B is 
Leu Arg Pro Gly
1
 wherein the Glycine residue is GlyNH₂ or Leu-Arg-Pro-NHCH₂CH₃;
when A is D-Phe-Cys-Phe, B is Lys-Thr-Cys-NHCH(CH₂OH)CHOH(CH₃) wherein the cysteine units are bound to each other by a cyclic disulphide;
when A is D-Phe-Cys-Tyr, B is 
Lys Val Cys Trp
1
 wherein the Tryptophan residue is Trp-NH₂ and the cysteine units are bound to each other by a cyclic disulphide;
when A is 
Pro Gln Gln Trp Phe Trp
1               5
 wherein the Proline residue is D-Pro and the Tryptophan residues at positions 4 and 6 are D-Trp, B is Met-NH₂
when A is Arg-D-Trp-N-methyl-Phe, B is Leu-Met-NH₂; and
when A is His, B is 
Ala Trp Phe Lys
1
 wherein the Phenylalanine residue is D-Phe and the Lysine residue is Lys-NH₂; wherein said peptides provide increased resistance to ionized sterilization, reactive radicals, and oxidation while maintaining a similar pharmacological effect as compared to analogous D-Tryptophan containing peptides not containing at least one D-2-alkylTryptophan.

5. The bioactive peptides of claim 4 wherein the lower alkyl group includes 1 to 3 carbon atoms.

6. The bioactive peptides of claim 5 wherein the alkyl group is methyl, ethyl, or isopropyl.

7. The bioactive peptides according to claim 4 wherein said peptide is 
Glu His Trp Ser Tyr Trp Leu Arg Pro Gly
1               5                   10
 wherein the Glutamic acid residue at position 1 is Pyro-Glu, the Tryptophan residue at position 6 is D-2-methyl-Trp, and the Glycine residue at position 10 is Gly-NH₂; 
Glu His Trp Ser Tyr Trp Leu Arg Pro
1               5
 wherein the Glutamic acid residue at position 1 is Pyro-Glu, the Tryptophan residue at position 6 is D-2-methyl-Trp, and the Proline residue at position 9 is Pro-NHCH₂CH₃; 
Pro Gln Gln Trp Phe Trp Trp Met
1               5
 wherein the Proline residue at position 1 is D-Proline, the Tryptophan residues at positions 4 and 6 are D-Trp, the Tryptophan residue at position 7 is D-2-ethyl-Trp, and the Methionine residue at position 8 is Met-NH₂; 
Arg Trp Phe Trp Leu Met
1               5
 wherein the Tryptophan residue at position 2 is D-Trp, the Phenylalanine residue at position 3 is N-methyl-Phe, the Tryptophan residue at position 4 is D-2-isopropyl-Trp, and the Methionine residue at position 6 is Met-NH₂; 
His Trp Ala Trp Phe Lys
1               5
 wherein the Tryptophan residue at position 2 is D-2-methyl-Trp, the Phenylalanine residue at position 5 is D-Phe, and the Lysine residue at position 6 is Lys-NH₂.

8. The bioactive peptides according to claim 4 wherein said peptide is 
Phe Cys Phe Trp Lys Thr Cys
1               5
 wherein the Phenylalanine residue at position 1 is D-Phe, the Tryptophan residue at position 4 is D-2-methyl-Trp, and the Cysteine residue at position 7 is Cys-NHCH(CH₂OH)CHOHCH₃ and wherein the cysteine units are bound to each other by means of a cyclic disulphide bond; or 
Phe Cys Tyr Trp Lys Val Cys Trp
1               5
 wherein the Phenylalanine residue at position 1 is D-Phe, the Tryptophan residue it position 4 is D-2-methyl-Trp, and the Tryptophan residue at position 8 is Trp-NH₂ wherein the cysteine units are bound to each other by means of a cyclic disulphide bond.

9. Bioactive peptides containing D-2-alkylTryptophan residues wherein said peptides are 
Glu His Trp Ser Tyr Trp Leu Arg Pro Gly
1               5                   10
 wherein the Glutamic acid residue at position 1 is Pyro-Glu, the Tryptophan residue at position 6 is D-2-methyl-Trp, and the Glycine residue at position 10 is Gly-NH₂; 
Glu His Trp Ser Tyr Trp Leu Arg Pro
1               5
 wherein the Glutamic Acid residue at position 1 is Pyro-Glu, the Tryptophan residue at position 6 is D-2-methyl-Trp and the Proline residue at position 9 is Pro-NHCH₂CH₃; 
Pro Gln Gln Trp Phe Trp Trp Met
1               5
 wherein the Proline residue at position 1 is D-proline, the Tryptophan residues at positions 4 and 6 are D-Trp, the Tryptophan residue at position 7 is D-2-ethyl-Trp and the Methionine residue at position 8 is Met-NH₂; 
Arg Trp Phe Trp Leu Met
1               5
 wherein the Tryptophan residue at position 2 is D-Trp, the Phenylalanine residue at position 3 is N-methyl-Phe, the Tryptophan residue at position 4 is D-2-isopropyl-Trp, and the Methionine residue at position 6 is Met-NH₂; 
Phe Cys Phe Trp Lys Thr Cys
1               5
 wherein the Phenylalanine residue at position 1 is D-Phe, the Tryptophan residue at position 4 is D-2-methyl-Trp, and the Cysteine residue at position 7 is Cys-NHCH(CH₂OH)CHOHCH₃ and wherein the cysteine units are bound to each other by means of a cyclic disulphide bond; 
Phe Cys Tyr Trp Lys Val Cys Trp
1               5
 wherein the Phenylalanine residue at position 1 is D-Phe, the Tryptophan residue at position 4 is D-2-methyl-Trp, and the Tryptophan residue at position 8 is Trp-NH₂ wherein the cysteine units are bound to each other by means of a cyclic disulphide bond; and 
His Trp Ala Trp Phe Lys
1               5
 wherein the Tryptophan residue at position 2 id D-2-methyl-Trp, the Phenylalanine residue at position 5 is D-Phe, and the Lysine residue at position 6 is Lys-NH₂.

10. A method for prolonging the pharmacological activity of a bioactive peptide containing one or more D-Tryptophan residues which comprises replacing at least one D-Tryptophan residue of said peptide with a D-2-alkylTryptophan residue so as to increase said peptides resistance to degradation by oxygen, reactive radicals and other reactive groups while maintaining a similar biological effect as compared to analogous bioactive peptides wherein the Tryptophan residue is not so replaced.

11. A method for preserving the pharmacological effectiveness of a bioactive peptide containing one or more Tryptophan residues which is to be sterilized or exposed to active or activated groups during manufacture which comprises replacing at least one Tryptophan residue of said peptide with a D-2-alkylTryptophan residue so as to increase the resistance of said peptide to the effects of sterilization or exposure to active or activated groups as compared to analogous bioactive peptides wherein the at least one Tryptophan residue is not so replaced.

12. The method of claim 11 wherein the bioactive peptide is sterilized by exposure to ionizing radiation.

13. The method of claim 11 wherein the peptide is exposed to the active or activated groups present in controlled delivery substances based on polylactic-polyglycolic acid polymers.

## Claims (Claims for the following Contracting State(s): ES, GR)

1. A process for the preparation of bioactive peptides containing D-Tryptophan residues wherein at least one D-Tryptophan residue is replaced with D-2-alkylTryptophan having a lower alkyl group substituted at the number 2 position, said D-2-alkylTryptophan providing increased oxidation resistance to the peptide while maintaining substantially the same pharmacological effect as analogous bioactive peptides in which the at least one Tryptophan residue is not replaced, characterised in that the peptides are synthesized by either the solution phase method or the solid phase method of conventional peptide synthesis.

2. A process according to claim 1 wherein in the bioactive peptides the lower alkyl group includes 1 to 3 carbons.

3. A process according to claim 2 wherein in the bioactive peptides the lower alkyl group is methyl, ethyl or isopropyl.

4. A process according to claim 1 wherein the bioactive peptides have formula A-D-2-alk-Trp-B, wherein alk is a lower alkyl group;
A is 
Glu His Trp Ser Tyr
 1               5
 wherein the Glutamic acid residue is pyroGlu; D-Phe-Cys-Phe; D-Phe-Cys-Tyr; 
Pro Gln Gln Trp Phe Trp
1               5
 wherein the Proline residue is D-Pro and the Tryptophan residues at positions 4 and 6 are D-Trp; Arg-D-Trp-N-methyl-Phe; or His; and
B is 
Leu Arg Pro Gly
1
 wherein the Glycine residue is Gly-NH₂; Leu-Arg-Pro-NHCH₂CH₃; Lys-Thr-Cys-NHCH(CH₂OH)CHOH(CH₃), 
Lys   Val   Cys   Trp
1
 wherein the Tryptophan residue is Trp-NH₂; Met-NH₂; Leu-Met-NH₂ or 
Ala Trp Phe Lys
1
 wherein the Phenylalanine residue is D-Phe and the Lysine residue is Lys-NH₂ provided that:
when A is 
Glu His Trp Ser Tyr
1               5
 wherein the Glutamic acid residue is pyroGlu, B is 
Leu Arg Pro Gly
1
 wherein the Glycine residue is GlyNH₂ or Leu-Arg-Pro-NHCH₂CH₃;
when A is D-Phe-Cys-Phe, B is Lys-Thr-Cys-NHCH(CH₂OH)CHOH(CH₃) wherein the cysteine units are bound to each other by a cyclic disulphide;
when A is D-Phe-Cys-Tyr, B is 
Lys Val Cys Trp
1
 wherein the Tryptophan residue is Trp-NH₂ and the cysteine units are bound to each other by a cyclic disulphide;
when A is 
Pro Gln Gln Trp Phe Trp
1               5
 wherein the Proline residue is D-Pro and the Tryptophan residues at positions 4 and 6 are D-Trp, B is Met-NH₂
when A is Arg-D-Trp-N-methyl-Phe, B is Leu-Met-NH₂; and
when A is His, B is 
Ala Trp Phe Lys
1
 wherein the Phenylalanine residue is D-Phe and the Lysine residue is Lys-NH₂; wherein said peptides provide increased resistance to ionized sterilization, reactive radicals, and oxidation while maintaining a similar pharmacological effect as compared to analogous D-Tryptophan containing peptides not containing at least one D-2-alkylTryptophan.

5. A process according to claim 4 wherein in the bioactive peptides the lower alkyl group includes 1 to 3 carbon atoms.

6. A process according to claim 5 wherein in the bioactive peptides the alkyl group is methyl, ethyl, or isopropyl.

7. A process according to claim 4 wherein the bioactive peptide is 
Glu His Trp Ser Tyr Trp Leu Arg Pro Gly
1               5                   10
 wherein the Glutamic acid residue at position 1 is Pyro-Glu, the Tryptophan residue at position 6 is D-2-methyl-Trp, and the Glycine residue at position 10 is Gly-NH₂; 
Glu His Trp Ser Tyr Trp Leu Arg Pro
1               5
 wherein the Glutamic acid residue at position 1 is Pyro-Glu, the Tryptophan residue at position 6 is D-2-methyl-Trp, and the Proline residue at position 9 is Pro-NHCH₂CH₃; 
Pro Gln Gln Trp Phe Trp Trp Met
1               5
 wherein the Proline residue at position 1 is D-Proline, the Tryptophan residues at positions 4 and 6 are D-Trp, the Tryptophan residue at position 7 is D-2-ethyl-Trp, and the Methionine residue at position 8 is Met-NH₂; 
Arg Trp Phe Trp Leu Met
1               5
 wherein the Tryptophan residue at position 2 is D-Trp, the Phenylalanine residue at position 3 is N-methyl-Phe, the Tryptophan residue at position 4 is D-2-isopropyl-Trp, and the Methionine residue at position 6 is Met-NH₂; 
His Trp Ala Trp Phe Lys
1               5
 wherein the Tryptophan residue at position 2 is D-2-methyl-Trp, the Phenylalanine residue at position 5 is D-Phe, and the Lysine residue at position 6 is Lys-NH₂.

8. A process according to claim 4 wherein the bioactive peptide is 
Phe Cys Phe Trp Lys Thr Cys
1               5
 wherein the Phenylalanine residue at position 1 is D-Phe, the Tryptophan residue at position 4 is D-2-methyl-Trp, and the Cysteine residue at position 7 is Cys-NHCH(CH₂OH)CHOHCH₃ and wherein the cysteine units are bound to each other by means of a cyclic disulphide bond; or 
Phe Cys Tyr Trp Lys Val Cys Trp
1               5
 wherein the Phenylalanine residue at position 1 is D-Phe, the Tryptophan residue at position 4 is D-2-methyl-Trp, and the Tryptophan residue at position 8 is Trp-NH₂ wherein the cysteine units are bound to each other by means of a cyclic disulphide bond.

9. A process according to claim 1 wherein the bioactive peptides contain D-2-alkylTryptophan residues wherein said peptides are 
Glu His Trp Ser Tyr Trp Leu Arg Pro Gly
1               5                   10
 wherein the Glutamic acid residue at position 1 is Pyro-Glu, the Tryptophan residue at position 6 is D-2-methyl-Trp, and the Glycine residue at position 10 is Gly-NH₂; 
Glu His Trp Ser Tyr Trp Leu Arg Pro
1               5
 wherein the Glutamic Acid residue at position 1 is Pyro-Glu, the Tryptophan residue at position 6 is D-2-methyl-Trp and the Proline residue at position 9 is Pro-NHCH₂CH₃; 
Pro Gln Gln Trp Phe Trp Trp Met
1               5
 wherein the Proline residue at position 1 is D-proline, the Tryptophan residues at positions 4 and 6 are D-Trp, the Tryptophan residue at position 7 is D-2-ethyl-Trp and the Methionine residue at position 8 is Met-NH₂; 
Arg Trp Phe Trp Leu Met
1               5
 wherein the Tryptophan residue at position 2 is D-Trp, the Phenylalanine residue at position 3 is N-methyl-Phe, the Tryptophan residue at position 4 is D-2-isopropyl-Trp, and the Methionine residue at position 6 is Met-NH₂; 
Phe Cys Phe Trp Lys Thr Cys
1               5
 wherein the Phenylalanine residue at position 1 is D-Phe, the Tryptophan residue at position 4 is D-2-methyl-Trp, and the Cysteine residue at position 7 is Cys-NHCH(CH₂OH)CHOHCH₃ and wherein the cysteine units are bound to each other by means of a cyclic disulphide bond; 
Phe Cys Tyr Trp Lys Val Cys Trp
 1               5
 wherein the Phenylalanine residue at position 1 is D-Phe, the Tryptophan residue at position 4 is D-2-methyl-Trp, and the Tryptophan residue at position 8 is Trp-NH₂ wherein the cysteine units are bound to each other by means of a cyclic disulphide bond; and 
His Trp Ala Trp Phe Lys
1               5
 wherein the Tryptophan residue at position 2 id D-2-methyl-Trp, the Phenylalanine residue at position 5 is D-Phe, and the Lysine residue at position 6 is Lys-NH₂.

## Patentansprüche (Patentansprüche für folgende(n) Vertragsstaat(en): AT, BE, CH, DE, DK, FR, GB, IT, LI, LU, NL, SE)

1. Bioaktive Peptide, die D-Tryptophan-Reste enthalten, worin wenigstens ein D-Tryptophan-Rest ersetzt ist durch D-2-alkyl-Tryptophan, das eine niedere Alkylgruppe hat, substituiert an der Position Nummer 2, wobei das D-2-alkylTryptophan dem Peptid eine erhöhte Sauerstoffbeständigkeit verleiht bei gleichzeitiger Aufrechterhaltung von im wesentlichen der gleichen pharmakologischen Wirkung wie bei analogen bioaktiven Peptiden, in denen der wenigstens eine Tryptophan-Rest nicht ersetzt ist.

2. Bioaktive Peptide nach Anspruch 1, worin die niedere Alkylgruppe 1 bis 3 Kohlenstoffatome enthält.

3. Bioaktive Peptide nach Anspruch 2, worin die niedere Alkylgruppe Methyl, Ethyl oder Isopropyl ist.

4. Bioaktive Peptide, gekennzeichnet durch die Formel A-D-2-alk-Trp-B, worin alk eine niedere Alkylgruppe ist;
A ist 
Glu His Trp Ser Tyr,
1               5
 worin der Glutaminsäure-Rest pyroGlu ist, D-Phe-Cys-Phe; D-Phe-Cys-Tyr; 
Pro Gln Gln Trp Phe Trp,
1               5
 worin der Prolin-Rest D-Pro ist und die Tryptophan-Reste an den Positionen 4 und 6 die Bedeutung D-Trp haben; Arg-D-Trp-N-methyl-Phe; oder His; und
B ist Leu Arg Pro Gly, worin der Glycin-Rest die Bedeutung Gly-NH₂ hat; Leu-Arg-Pro-NHCH₂CH₃; Lys-Thr-Cys-NHCH(CH₂OH)CHOH(CH₃), 
Lys Val Cys Trp,
1
 worin der Tryptophan-Rest Trp-NH₂ ist; Met-NH₂; Leu-Met-NH₂ oder 
Ala Trp Phe Lys,
  1
 worin der Phenylalanin-Rest die Bedeutung D-Phe hat, und der Lysin-Rest die Bedeutung Lys-NH₂ hat, mit der Maßgabe, daß:
wenn A die Bedeutung 
Glu His Trp Ser Tyr
1               5
 hat, worin der Glutaminsäure-Rest pyroGlu ist, B die Bedeutung 
Leu Arg Pro Gly
1
 hat, worin der Glycin-Rest GlyNH₂ ist, oder Leu-Arg-Pro-NHCH₂CH₃;
wenn A die Bedeutung D-Phe-Cys-Phe hat, hat B die Bedeutung Lys-Thr-Cys-NHCH(CH₂OH)CHOH(CH₃), worin die Cystein-Einheiten durch ein cyclisches Disulfid aneinander gebunden sind;
wenn A die Bedeutung D-Phe-Cys-Tyr hat, hat B die Bedeutung 
Lys Val Cys Trp,
 1
 worin der Tryptophan-Rest Trp-NH₂ ist, und die Cystein-Einheiten sind durch ein cyclisches Disulfid aneinander gebunden;
wenn A die Bedeutung 
Pro Gln Gln Trp Phe Trp
1               5
 hat, worin der Prolinrest D-Pro ist und die Tryptophan-Reste an den Positionen 4 und 6 sind D-Trp, hat B die Bedeutung Met-NH₂;
wenn A die Bedeutung Arg-D-Trp-N-methyl-Phe hat, hat B die Bedeutung Leu-Met-NH₂; und
wenn A die Bedeutung His hat, hat B die Bedeutung 
Ala Trp Phe Lys,
 1
 worin der Phenylalanin-Rest D-Phe ist und der Lysin-Rest ist Lys-NH₂; wobei die Peptide eine erhöhte Sauerstoffbeständigkeit verleihen gegenüber Ionisierungssterilisation, reaktiven Radikalen und Oxidation bei gleichzeitiger Aufrechterhaltung einer ähnlichen pharmakologischen Wirkung im Vergleich mit analogen D-Tryptophan enthaltenden Peptiden, die nicht wenigstens ein D-2-alkylTryptophan enthalten.

5. Bioaktive Peptide nach Anspruch 4, worin die niedere Alkylgruppe 1 bis 3 Kohlenstoffatome einschließt.

6. Bioaktive Peptide nach Anspruch 5, worin die niedere Alkylgruppe Methyl, Ethyl oder Isopropyl ist.

7. Bioaktive Peptide nach Anspruch 4, worin das Peptid 
Glu His Trp Ser Tyr Trp Leu Arg Pro Gly
1               5                   10
 ist, worin der Glutaminsäure-Rest an Position 1 die Bedeutung Pyro-Glu hat, der Tryptophan-Rest an Position 6 ist D-2-methyl-Trp, und der Glycin-Rest an Position 10 ist Gly-NH₂; 
Glu His Trp Ser Tyr Trp Leu Arg Pro,
1               5
 worin der Glutaminsäure-Rest an Position 1 die Bedeutung Pyro-Glu hat, der Tryptophan-Rest an Position 6 ist D-2-methyl-Trp und der Prolin-Rest an Position 9 ist Pro-NHCH₂CH₃: 
Pro Gln Gln Trp Phe Trp Trp Met,
1               5
 worin der Prolin-Rest an Position 1 die Bedeutung D-Prolin hat, die Tryptophan-Reste an den Positionen 4 und 6 sind D-Trp, der Tryptophan-Rest an Position 7 ist D-2-ethyl-Trp und der Methionin-Rest an Position 8 ist Met-NH₂; 
Arg Trp Phe Trp Leu Met,
1               5
 worin der Tryptophan-Rest an Position 2 die Bedeutung D-Trp hat, der Phenylalanin-Rest an Position 3 ist N-methyl-Phe, der Tryptophan-Rest an Position 4 ist D-2-isopropyl-Trp, und der Methionin-Rest an Position 6 ist Met-NH₂; 
His Trp Ala Trp Phe Lys,
 1                   5
 worin der Tryptophan-Rest an Position 2 die Bedeutung D-2-methyl-Trp hat, der Phenylalanin-Rest an Position 5 ist D-Phe, und der Lysin-Rest an Position 6 ist Lys-NH₂.

8. Bioaktive Peptide nach Anspruch 4, worin das Peptid 
Phe Cys Phe Trp Lys Thr Cys
1               5
 ist, worin der Phenylalanin-Rest an Position 1 die Bedeutung D-Phe hat, der Tryptophan-Rest an Position 4 ist D-2-methyl-Trp, und der Cystein-Rest an Position 7 ist Cys-NHCH(CH₂OH)CHOHCH₃, und worin die Cystein-Einheiten mittels eines cyclischen Diusulfides aneinander gebunden sind; oder 
Phe Cys Tyr Trp Lys Val Cys Trp,
 1               5
 worin der Phenylalanin-Rest an Position 1 die Bedeutung D-Phe hat, der Tryptophan-Rest an Position 4 ist D-2-methyl-Trp, und der Tryptophan-Rest an Position 8 ist Trp-NH₂, worin die Cystein-Einheiten mittels einer cyclischen Diusulfidbindung aneinander gebunden sind.

9. Bioaktive Peptide, die D-2-alkylTryptophan-Reste enthalten, worin die Peptide die Bedeutung 
Glu His Trp Ser Tyr Trp Leu Arg Pro Gly
1               5                   10
 haben, worin der Glutaminsäure-Rest an Position 1 die Bedeutung Pyro-Glu hat, der Tryptophan-Rest an Position 6 ist D-2-methyl-Trp, und der Glycin-Rest an Position 10 ist Gly-NH₂; 
Glu His Trp Ser Tyr Trp Leu Arg Pro,
1               5
 worin der Glutaminsäure-Rest an Position 1 die Bedeutung Pyro-Glu hat, der Tryptophan-Rest an Position 6 ist D-2-methyl-Trp, und der Prolin-Rest an Position 9 ist Pro-NHCH₂CH₃; 
Pro Gln Gln Trp Phe Trp Trp Met,
1               5
 worin der Prolinrest an Position 1 die Bedeutung D-Prolin hat, die Tryptophan-Reste an den Positionen 4 und 6 sind D-Trp, der Tryptophan-Rest an Position 7 ist D-2-ethyl-Trp, und der Methionin-Rest an Position 8 ist Met-NH₂; 
Arg Trp Phe Trp Leu Met,
1               5
 worin der Tryptophan-Rest an Position 2 die Bedeutung D-Trp hat, der Phenylalanin-Rest an Position 3 ist N-methyl-Phe, der Tryptophan-Rest an Position 4 ist D-2-isopropyl-Trp, und der Methionin-Rest an Position 6 ist Met-NH₂; 
Phe Cys Phe Trp Lys Thr Cys,
 1               5
 worin der Phenylalanin-Rest an Position 1 die Bedeutung D-Phe hat, der Tryptophan-Rest an Position 4 ist D-2-methyl-Trp, und der Cystein-Rest an Position 7 ist Cys-NHCH(CH₂OH)CHOHCH₃, und worin die Cystein-Einheiten mittels einer cyclischen Disulfidbindung aneinander gebunden sind; 
Phe Cys Tyr Trp Lys Val Cys Trp,
 1               5
 worin der Phenylalanin-Rest an Position 1 die Bedeutung D-Phe hat, der Tryptophan-Rest an Position 4 ist D-2-methyl-Trp, und der Tryptophan-Rest an Position 8 ist Trp-NH₂, worin die Cystein-Einheiten durch eine cyclische Disulfidbindung aneinander gebunden sind; und 
His Trp Ala Trp Phe Lys,
1               5
 worin der Tryptophan-Rest an Position 2 die Bedeutung D-2-methyl-Trp hat, der Phenylalanin-Rest an Position 5 ist D-Phe, und der Lysin-Rest an Position 6 ist Lys-NH₂.

10. Verfahren zur Verlängerung der pharmakologischen Wirksamkeit eines bioaktiven Peptides, das einen oder mehrere D-Tryptophan-Reste enthält, wobei das Verfahren das Ersetzen von wenigstens einem D-Tryptophan-Rest des Peptides durch einen D-2-alkylTryptophan-Rest umfaßt, so daß die Peptidbeständigkeit gegen Abbau durch Sauerstoff, reaktive Radikale und andere Gruppen erhöht wird, während eine ähnliche biologische Wirkung aufrechterhalten wird im Vergleich mit analogen bioaktiven Peptiden, bei denen der Tryptophan-Rest nicht so ersetzt wurde.

11. Verfahren zur Erhaltung der pharmakologischen Wirksamkeit eines bioaktiven Peptides, das einen oder mehrere Tryptophan-Reste enthält, das zu sterilisieren ist oder während der Herstellung aktiven oder aktivierten Gruppen ausgesetzt ist, wobei das Verfahren umfaßt das Ersetzen von wenigstens einem Tryptophan-Rest des Peptides mit einem D-2-alkylTryptophan-Rest, so daß sich die Beständigkeit des Peptides gegen die Wirkungen der Sterilisation oder dem Aussetzen gegenüber aktiven oder aktivierten Gruppen erhöht im Vergleich mit analogen bioaktiven Peptiden, bei denen wenigstens ein Tryptophan-Rest nicht so ersetzt wurde.

12. Verfahren nach Anspruch 11, worin das bioaktive Peptid sterilisiert wird, indem es ionisierender Strahlung ausgesetzt wird.

13. Verfahren nach Anspruch 11, worin das Peptid den aktiven oder aktivierten Gruppen ausgesetzt wird, die in gesteuert freisetzenden Substanzen auf Basis von Polymilchsäure-Polyglycolsäure-Polymeren vorhanden sind.

## Patentansprüche (Patentansprüche für folgende(n) Vertragsstaat(en): ES, GR)

1. Verfahren zur Herstellung von bioaktiven Peptiden, die D-Tryptophan-Reste enthalten, worin wenigstens ein D-Tryptophan-Rest ersetzt ist durch D-2-alkylTryptophan, das eine niedere Alkylgruppe hat, substituiert an der Position Nummer 2, wobei das D-2-alkylTryptophan dem Peptid eine erhöhte Sauerstoffbeständigkeit verleiht bei gleichzeitiger Aufrechterhaltung von im wesentlichen der gleichen pharmakologischen Wirkung wie bei analogen bioaktiven Peptiden, in denen der wenigstens eine Tryptophan-Rest nicht ersetzt ist, dadurch gekennzeichnet, daß die Peptide entweder nach dem Lösungsphasenverfahren oder nach dem Festphasenverfahren der üblichen Peptidsynthesen synthetisiert werden.

2. Verfahren nach Anspruch 1, worin die niedere Alkylgruppe 1 bis 3 Kohlenstoffatome enthält.

3. Verfahren nach Anspruch 2, worin die niedere Alkylgruppe Methyl, Ethyl oder Isopropyl ist.

4. Verfahren nach Anspruch 1, worin die bioaktiven Peptide die Formel A-D-2-alk-Trp-B haben, worin alk eine niedere Alkylgruppe ist;
A ist 
Glu His Trp Ser Tyr,
1               5
 worin der Glutaminsäure-Rest pyroGlu ist, D-Phe-Cys-Phe; D-Phe-Cys-Tyr; 
Pro Gln Gln Trp Phe Trp,
1               5
 worin der Prolin-Rest D-Pro ist und die Tryptophan-Reste an den Positionen 4 und 6 die Bedeutung D-Trp haben; Arg-D-Trp-N-methyl-Phe; oder His; und
B ist Leu Arg Pro Gly, worin der Glycin-Rest die Bedeutung Gly-NH₂ hat; Leu-Arg-Pro-NHCH₂CH₃; Lys-Thr-Cys-NHCH(CH₂OH)CHOH(CH₃), 
Lys Val Cys Trp,
  1
 worin der Tryptophan-Rest Trp-NH₂ ist; Met-NH₂; Leu-Met-NH₂ oder 
Ala Trp Phe Lys,
 1
 worin der Phenylalanin-Rest die Bedeutung D-Phe hat, und der Lysin-Rest die Bedeutung Lys-NH₂ hat, mit der Maßgabe, daß:
wenn A die Bedeutung 
Glu His Trp Ser Tyr
1               5
 hat, worin der Glutaminsäure-Rest pyroGlu ist, B die Bedeutung 
Leu Arg Pro Gly
1
 hat, worin der Glycin-Rest GlyNH₂ ist, oder Leu-Arg-Pro-NHCH₂CH₃;
wenn A die Bedeutung D-Phe-Cys-Phe hat, hat B die Bedeutung Lys-Thr-Cys-NHCH(CH₂OH)CHOH(CH₃), worin die Cystein-Einheiten durch ein cyclisches Disulfid aneinander gebunden sind;
wenn A die Bedeutung D-Phe-Cys-Tyr hat, hat B die Bedeutung 
Lys Val Cys Trp,
1
 worin der Tryptophan-Rest Trp-NH₂ ist, und die Cystein-Einheiten sind durch ein cyclisches Disulfid aneinander gebunden;
wenn A die Bedeutung 
Pro Gln Gln Trp Phe Trp
1               5
 hat, worin der Prolinrest D-Pro ist und die Tryptophan-Reste an den Positionen 4 und 6 sind D-Trp, hat B die Bedeutung Met-NH₂;
wenn A die Bedeutung Arg-D-Trp-N-methyl-Phe hat, hat B die Bedeutung Leu-Met-NH₂; und
wenn A die Bedeutung His hat, hat B die Bedeutung 
Ala Trp Phe Lys,
 1
 worin der Phenylalanin-Rest D-Phe ist und der Lysin-Rest ist Lys-NH₂; wobei die Peptide eine erhöhte Sauerstoffbeständigkeit verleihen gegenüber Ionisierungssterilisation, reaktiven Radikalen und Oxidation bei gleichzeitiger Aufrechterhaltung einer ähnlichen pharmakologischen Wirkung im Vergleich mit analogen D-Tryptophan enthaltenden Peptiden, die nicht wenigstens ein D-2-alkylTryptophan enthalten.

5. Verfahren nach Anspruch 4, worin die niedere Alkylgruppe 1 bis 3 Kohlenstoffatome einschließt.

6. Verfahren nach Anspruch 5, worin die niedere Alkylgruppe Methyl, Ethyl oder Isopropyl ist.

7. Verfahren nach Anspruch 4, worin das bioaktive Peptid 
Glu His Trp Ser Tyr Trp Leu Arg Pro Gly
1               5                   10
 ist, worin der Glutaminsäure-Rest an Position 1 die Bedeutung Pyro-Glu hat, der Tryptophan-Rest an Position 6 ist D-2-methyl-Trp, und der Glycin-Rest an Position 10 ist Gly-NH₂; 
Glu His Trp Ser Tyr Trp Leu Arg Pro,
1               5
 worin der Glutaminsäure-Rest an Position 1 die Bedeutung Pyro-Glu hat, der Tryptophan-Rest an Position 6 ist D-2-methyl-Trp und der Prolin-Rest an Position 9 ist Pro-NHCH₂CH₃; 
Pro Gln Gln Trp Phe Trp Trp Met,
 1               5
 worin der Prolin-Rest an Position 1 die Bedeutung D-Prolin hat, die Tryptophan-Reste an den Positionen 4 und 6 sind D-Trp, der Tryptophan-Rest an Position 7 ist D-2-ethyl-Trp und der Methionin-Rest an Position 8 ist Met-NH₂; 
Arg Trp Phe Trp Leu Met,
 1               5
 worin der Tryptophan-Rest an Position 2 die Bedeutung D-Trp hat, der Phenylalanin-Rest an Position 3 ist N-methyl-Phe, der Tryptophan-Rest an Position 4 ist D-2-isopropyl-Trp, und der Methionin-Rest an Position 6 ist Met-NH₂; 
His Trp Ala Trp Phe Lys,
1               5
 worin der Tryptophan-Rest an Position 2 die Bedeutung D-2-methyl-Trp hat, der Phenylalanin-Rest an Position 5 ist D-Phe, und der Lysin-Rest an Position 6 ist Lys-NH₂.

8. Verfahren nach Anspruch 4, worin das bioaktive Peptid 
Phe Cys Phe Trp Lys Thr Cys
 1               5
 ist, worin der Phenylalanin-Rest an Position 1 die Bedeutung D-Phe hat, der Tryptophan-Rest an Position 4 ist D-2-methyl-Trp, und der Cystein-Rest an Position 7 ist Cys-NHCH(CH₂OH)CHOHCH₃, und worin die Cystein-Einheiten mittels eines cyclischen Diusulfides aneinander gebunden sind; oder 
Phe Cys Tyr Trp Lys Val Cys Trp,
 1              5
 worin der Phenylalanin-Rest an Position 1 die Bedeutung D-Phe hat, der Tryptophan-Rest an Position 4 ist D-2-methyl-Trp, und der Tryptophan-Rest an Position 8 ist Trp-NH₂, worin die Cystein-Einheiten mittels einer cyclischen Diusulfidbindung aneinander gebunden sind.

9. Verfahren nach Anspruch 1, worin die bioaktiven Peptide D-2-alkylTryptophan-Reste enthalten, worin die Peptide die Bedeutung 
Glu His Trp Ser Tyr Trp Leu Arg Pro Gly
1               5                   10
 haben, worin der Glutaminsäure-Rest an Position 1 die Bedeutung Pyro-Glu hat, der Tryptophan-Rest an Position 6 ist D-2-methyl-Trp, und der Glycin-Rest an Position 10 ist Gly-NH₂; 
Glu His Trp Ser Tyr Trp Leu Arg Pro,
1               5
 worin der Glutaminsäure-Rest an Position 1 die Bedeutung Pyro-Glu hat, der Tryptophan-Rest an Position 6 ist D-2-methyl-Trp, und der Prolin-Rest an Position 9 ist Pro-NHCH₂CH₃; 
Pro Gln Gln Trp Phe Trp Trp Met,
1               5
 worin der Prolinrest an Position 1 die Bedeutung D-Prolin hat, die Tryptophan-Reste an den Positionen 4 und 6 sind D-Trp, der Tryptophan-Rest an Position 7 ist D-2-ethyl-Trp, und der Methionin-Rest an Position 8 ist Met-NH₂; 
Arg Trp Phe Trp Leu Met,
 1                   5
 worin der Tryptophan-Rest an Position 2 die Bedeutung D-Trp hat, der Phenylalanin-Rest an Position 3 ist N-methyl-Phe, der Tryptophan-Rest an Position 4 ist D-2-isopropyl-Trp, und der Methionin-Rest an Position 6 ist Met-NH₂; 
Phe Cys Phe Trp Lys Thr Cys,
 1              5
 worin der Phenylalanin-Rest an Position 1 die Bedeutung D-Phe hat, der Tryptophan-Rest an Position 4 ist D-2-methyl-Trp, und der Cystein-Rest an Position 7 ist Cys-NHCH(CH₂OH)CHOHCH₃, und worin die Cystein-Einheiten mittels einer cyclischen Disulfidbindung aneinander gebunden sind; 
Phe Cys Tyr Trp Lys Val Cys Trp,
1               5
 worin der Phenylalanin-Rest an Position 1 die Bedeutung D-Phe hat, der Tryptophan-Rest an Position 4 ist D-2-methyl-Trp, und der Tryptophan-Rest an Position 8 ist Trp-NH₂, worin die Cystein-Einheiten durch eine cyclische Disulfidbindung aneinander gebunden sind; und 
His Trp Ala Trp Phe Lys,
1               5
 worin der Tryptophan-Rest an Position 2 die Bedeutung D-2-methyl-Trp hat, der Phenylalanin-Rest an Position 5 ist D-Phe, und der Lysin-Rest an Position 6 ist Lys-NH₂.

## Revendications (Revendications pour l'(les) Etat(s) contractant(s) suivant(s): AT, BE, CH, DE, DK, FR, GB, IT, LI, LU, NL, SE)

1. Peptides bioactifs contenant des résidus D-Tryptophane dans lesquels au moins un résidu D-Tryptophane est remplacé par D-2-alkylTryptophane possédant un groupe alkyle inférieur substitué à la position numéro 2, ledit D-2-alkylTryptophane conférant au peptide une résistance accrue à l'oxydation tout en maintenant pour l'essentiel le même effet pharmacologique que les peptides bioactifs analogues dans lesquels au moins un résidu Tryptophane n'est pas remplacé.

2. Les peptides bioactifs selon la revendication 1 dans lesquels le groupe alkyle inférieur comprend de un à trois atomes de carbone.

3. Les peptides bioactifs selon la revendication 2 dans lesquels le groupe alkyle inférieur est méthyle, éthyle, ou isopropyle.

4. Peptides bioactifs caractérisés par la formule A-D-2-alk-Trp-B, dans laquelle alk est un groupe alkyle inférieur;
A est 
Glu His Trp Ser Tyr
1               5
 dans lequel le résidu acide Glutamique est pyroGlu; D-Phe-Cys-Phe; D-Phe-Cys-Tyr; 
Pro Gln Gln Trp Phe Trp
1               5
 dans lequel le résidu Proline est D-Pro et les résidus Tryptophane aux positions 4 et 6 sont D-Trp; Arg-D-Trp-N-méthyl-Phe; ou His; et
B est 
Leu Arg Pro Gly,
1
 dans lequel le résidu Glycine est Gly-NH₂; Leu-Arg-Pro-NHCN₂CH₃; Lys-Thr-Cys-NHCH(CH₂OH)CHOH(CH₃), 
Lys Val Cys Trp
1
 dans lequel le résidu Tryptophane est Trp-NH₂; Met-NH₂; Leu-Met-NH₂ ou 
Ala Trp Phe Lys
1
 dans lequel le résidu Phénylalanine est D-Phe et le résidu Lysine est Lys-NH₂, à condition que : lorsque A est 
Glu His Trp Ser Tyr,
1               5
 dans lequel le résidu acide Glutamique est pyroGlu, B est 
Leu Arg Pro Gly
1
 dans lequel le résidu Glycine est GlyNH₂ ou Leu-Arg-Pro-NHCH₂CH₃;
lorsque A est D-Phe-Cys-Phe, B est Lys-Thr-Cys-NHCH(CH₂OH)CHOH(CH₃) dans lequel les résidus Cystéine sont liés l'un à l'autre par un pont disulfure cyclique;
lorsque A est D-Phe-Cys-Tyr, B est 
Lys Val Cys Trp
1
 dans lequel le résidu Tryptophane est Trp-NH₂ et les résidus Cystéine sont liés l'un à l'autre par un pont disulfure cyclique;
lorsque A est 
Pro Gln Gln Trp Phe Trp
1               5
 dans lequel le résidu Proline est D-Pro et les résidus Tryptophane aux positions 4 et 6 sont des D-Trp, B est Met-NH₂ lorsque A est Arg-D-Trp-N-méthyl-Phe, B est Leu-Met-NH₂; et lorsque A est His, B est 
Ala Trp Phe Lys
1
 dans lequel le résidu Phénylalanine est D-Phe et le résidu Lysine est Lys-NH₂; dans lesquels lesdits peptides procurent une résistance accrue à la stérilisation par ionisation, aux radicaux réactifs, et à l'oxydation tout en maintenant un effet pharmacologique similaire comparé aux peptides analogues contenant du D-Tryptophane et ne contenant pas au moins un D-2-alkylTryptophane.

5. Les peptides bioactifs selon la revendication 4 dans lesquels le groupe alkyle inférieur contient de un à trois atomes de carbone.

6. Les peptides bioactifs selon la revendication 5 dans lesquels le groupement alkyle est méthyle, éthyle ou isopropyle.

7. Les peptides bioactifs selon la revendication 4 dans lesquels le dit peptide bioactif est 
Glu His Trp Ser Tyr Trp Leu Arg Pro Gly10
1               5
 dans lequel le résidu acide Glutamique à la position 1 est Pyro-Glu, le résidu Tryptophane à la position 6 est D-2-méthyl-Trp, et le résidu Glycine à la position 10 est Gly-NH₂; 
Glu His Trp Ser Tyr Trp Leu Arg Pro
1               5
 dans lequel le résidu acide Glutamique à la position 1 est Pyro-Glu, le résidu Tryptophane à la position 6 est D-2-méthyl-Trp, et le résidu Proline à la position 9 est Pro-NHCH₂CH₃; 
Pro Gln Gln Trp Phe Trp Trp Met
1               5
 dans lequel le résidu Proline à la position 1 est D-Proline, les résidus Tryptophane aux positions 4 et 6 sont D-Trp, le résidu Tryptophane à la position 7 est D-2-éthyl-Trp, et le résidu Méthionine à la position 8 est Met-NH₂; 
Arg Trp Phe Trp Leu Met
1               5
 dans lequel le résidu Tryptophane à la position 2 est D-Trp, le résidu Phénylalanine à la position 3 est N-méthyl-Phe, le résidu Tryptophane à la position 4 est D-2-isoPropyl-Trp, et le résidu Méthionine à la position 6 est Met-NH₂; 
His Trp Ala Trp Phe Lys
1               5
 dans lequel le résidu Tryptophane à la position 2 est D-2-méthyl-Trp, le résidu Phénylalanine à la position 5 est D-Phe, et le résidu Lysine à la position 6 est Lys-NH₂.

8. Les peptides bioactifs selon la revendication 4 dans lesquels le dit peptide bioactif est 
Phe Cys Phe Trp Lys Thr Cys
1               5
 dans lequel le résidu Phénylalaline à la position 1 est D-Phe, le résidu Tryptophane à la position 4 est D-3-méthyl-Trp, et le résidu Cystéine à la position 7 est Cys-NHCH(CH₂OH)CHOHCH₃ et dans lequel les résidus Cystéine sont liés l'un à l'autre par un pont disulfure cyclique; ou 
Phe Cys Tyr Trp Lys Val Cys Trp
1               5
 dans lequel le résidu Phénylalanine à la position 1 est D-Phe, le résidu Tryptophane à la position 4 est D-2-méthyl-Trp, et le résidu Tryptophane à la position 8 est Trp-NH₂, dans lequel les résidus Cystéine sont liées l'un à l'autre par un pont disulfure cyclique.

9. Peptides bioactifs contenant des résidus D-2-alkylTryptophane dans lesquels les dits peptides sont 
Glu His Trp Ser Tyr Trp Leu Arg Pro Gly
1               5                   10
 dans lesquels le résidu acide Glutamique à la position 1 est Pyro-Glu, le résidu Tryptophane à la position 6 est D-2-méthyl-Trp, et le résidu Glycine à la position 10 est Gly-NH₂; 
Glu His Trp Ser Tyr Trp Leu Arg Pro'
1               5
 dans lequel le résidu acide Glutamique à la position 1 est Pyro-Glu, le résidu Tryptophane à la position 6 est D-2-méthyl-Trp et le résidu Proline à la position 9 est Pro-NHCH₂CH₃; 
Pro Gln Gln Trp Phe Trp Trp Met
1               5
 dans lequel le résidu Proline à la position 1 est D-Proline, les résidus Tryptophane aux positions 4 et 6 sont D-Trp, le résidu Tryptophane à la position 7 est D-2-éthyl-Trp et le résidu Méthionine à la position 8 est Met-NH₂; 
Arg Trp Phe Trp Leu Met
1               5
 dans lequel le résidu Tryptophane à la position 2 est D-Trp, le résidu Phénylalanine à la position 3 est N-méthyl-Phe, le résidu Tryptophane à la position 4 est D-2-isoPropyl-Trp, et le résidu Méthionine à la position 6 est Met-NH₂; 
Phe Cys Phe Trp Lys Thr Cys
1               5
 dans lequel le résidu Phénylalanine à la position 1 est D-Phe, le résidu Tryptophane à la position 4 est D-2-méthyl-Trp, et le résidu Cystéine à la position 7 est Cys-NHCH(CH₂OH)CHOHCH₃ et dans lequel les résidus Cystéine sont liés l'un à l'autre par un pont disulfure cyclique; 
Phe Cys Tyr Trp Lys Val Cys Trp
1               5
 dans lequel le résidu Phénylalanine à la position 1 est D-Phe, le résidu Tryptophane à la position 4 est D-2-méthyl-Trp, et le résidu Tryptophane à la position 8 est Trp-NH₂ dans lesquels les unités Cystéine sont liées l'une à l'autre par un pont disulfure cyclique; et 
His Trp Ala Trp Phe Lys
1               5
 dans lequel le résidu Tryptophane à la position 2 est D-2-méthyl-Trp, le résidu Phénylalanine à la position 5 est D-Phe, et le résidu Lysine à la position 6 est Lys-NH₂.

10. Méthode permettant de Prolonger l'activité pharmacologique d'un peptide bioactif contenant un ou plusieurs résidus D-Tryptophane, qui consiste à remplacer au moins un résidu D-Tryptophane du dit peptide par un résidu D-2-alkylTryptophane de manière à augmenter la résistance du dit peptide à la dégradation par l'oxygène, les radicaux réactifs et autres groupements réactifs tout en maintenant un effet biologique similaire comparativement à des peptides bioactifs analogues dans lesquels le résidu Tryptophane n'est pas ainsi remplacé.

11. Méthode permettant de conserver l'efficacité pharmacologique d'un peptide bioactif contenant un ou plusieurs résidus Tryptophane et devant être stérilisé ou exposé à des groupements actifs ou activés pendant sa fabrication, qui consiste à remplacer au moins un résidu D-Tryptophane du dit peptide par un résidu D-2-alkylTryptophane de manière à augmenter la résistance du dit peptide aux effets de la stérilisation ou de l'exposition à des groupements actifs ou activés comparativement à des peptides bioactifs analogues dans lesquels aucun résidu Tryptophane n'est ainsi remplacé.

12. Méthode selon la revendication 11 dans laquelle le peptide bioactif est stérilisé par exposition à des radiations ionisantes.

13. Méthode selon la revendication 11, dans laquelle le peptide est exposé à des groupements actifs ou activés présents dans des substances à libération contrôlée à base de polymères d'acides polylactique-polyglycolique.

## Revendications (Revendications pour l'(les) Etat(s) contractant(s) suivant(s): ES, GR)

1. Procédé de préparation de peptides bioactifs contenant des résidus D-Tryptophane dans lesquels au moins un résidu D-Tryptophane est remplacé par D-2-alkylTryptophane possédant un groupe alkyle inférieur substitué à la position numéro 2, ledit D-2-alkylTryptophane conférant au peptide une résistance accrue à l'oxydation tout en maintenant pour l'essentiel le même effet pharmacologique que les peptides bioactifs analogues dans lesquels au moins un résidu Tryptophane n'est pas remplacé, caractérisé en ce que les peptides sont synthètisés selon la méthode conventionnelle de synthèse, soit en phase soluble soit en phase solide.

2. Procédé selon la revendication 1 dans lequel, dans les peptides bioactifs, le groupe alkyle inférieur comprend de 1 à 3 carbones.

3. Procédé selon la revendication 2 dans lequel, dans les peptides bioactifs, le groupe alkyle inférieur est méthyle, éthyle ou isoPropyle.

4. Procédé selon la revendication 1 dans lequel les peptides bioactifs ont la formule A-D-2-alk-Trp-B dans laquelle alk est un groupe alkyle inférieur;
A est 
Glu His Trp Ser Tyr
1               5
 dans lequel le résidu acide Glutamique est pyroGlu; D-Phe-Cys-Phe; D-Phe-Cys-Tyr; 
Pro Gln Gln Trp Phe Trp
1               5
 dans lequel le résidu Proline est D-Pro et les résidus Tryptophane aux positions 4 et 6 sont D-Trp; Arg-D-Trp-N-méthyl-Phe; ou His; et
B est 
Leu Arg Pro Gly,
 1
 dans lequel le résidu Glycine est Gly-NH₂; Leu-Arg-Pro-NHCN₂CH₃; Lys-Thr-Cys-NHCH(CH₂OH)CHOH(CH₃), 
Lys Val Cys Trp
1
 dans lequel le résidu Tryptophane est Trp-NH₂; Met-NH₂; Leu-Met-NH₂ ou 
Ala Trp Phe Lys
1
 dans lequel le résidu Phénylalanine est D-Phe et le résidu Lysine est Lys-NH₂, à condition que :
lorsque A est 
Glu His Trp Ser Tyr,
 1              5
 dans lequel le résidu acide Glutamique est pyroGlu, B est 
Leu Arg Pro Gly
1
 dans lequel le résidu Glycine est GlyNH₂ ou Leu-Arg-Pro-NHCH₂CH₃;
lorsque A est D-Phe-Cys-Phe, B est Lys-Thr-Cys-NHCH(CH₂OH)CHOH(CH₃) dans lequel les résidus Cystéine sont liés l'un à l'autre par un pont disulfure cyclique;
lorsque A est D-Phe-Cys-Tyr, B est 
Lys Val Cys Trp
1
 dans lequel le résidu Tryptophane est Trp-NH₂ et les résidus Cystéine sont liés l'un à l'autre par un pont disulfure cyclique;
lorsque A est Pro₁ Gln Gln Trp Phe₅ Trp dans lequel le résidu Proline est D-Pro et les résidus Tryptophane aux positions 4 et 6 sont des D-Trp, B est Met-NH₂ lorsque A est Arg-D-Trp-N-méthyl-Phe, B est Leu-Met-NH₂; et lorsque A est His, B est 
Ala Trp Phe Lys
1
 dans lequel le résidu Phénylalanine est D-Phe et le résidu Lysine est Lys-NH₂; dans lesquels lesdits peptides Procurent une résistance accrue à la stérilisation par ionisation, aux radicaux réactifs, et à l'oxydation tout en maintenant un effet pharmacologique similaire comparé aux peptides analogues contenant du D-Tryptophane et ne contenant pas au moins un D-2-alkylTryptophane.

5. Procédé selon la revendication 4 dans lequel, dans les peptides bioactifs, le groupe alkyle inférieur comporte 1 à 3 atomes de carbone.

6. Procédé selon la revendication 5 dans lequel, dans les peptides bioactifs, le groupe alkyle est méthyle, éthyle ou isoPropyle.

7. Procédé selon la revendication 4 dans lequel le peptide bioactif est 
Glu His Trp Ser Tyr Trp Leu Arg Pro Gly
1               5                   10
 dans lequel le résidu acide Glutamique à la position 1 est Pyro-Glu, le résidu Tryptophane à la position 6 est D-2-méthyl-Trp, et le résidu Glycine à la position 10 est Gly-NH₂; 
Glu His Trp Ser Tyr Trp Leu Arg Pro
 1               5
 dans lequel le résidu acide Glutamique à la position 1 est Pyro-Glu, le résidu Tryptophane à la position 6 est D-2-méthyl-Trp, et le résidu Proline à la position 9 est Pro-NHCH₂CH₃; 
Pro Gln Gln Trp Phe Trp Trp Met
1               5
 dans lequel le résidu Proline à la position 1 est D-Proline, les résidus Tryptophane aux positions 4 et 6 sont D-Trp, le résidu Tryptophane à la position 7 est D-2-éthyl-Trp, et le résidu Méthionine à la position 8 est Met-NH₂; 
Arg Trp Phe Trp Leu Met
 1               5
 dans lequel le résidu Tryptophane à la position 2 est D-Trp, le résidu Phénylalanine à la position 3 est N-méthyl-Phe, le résidu Tryptophane à la position 4 est D-2-isoPropyl-Trp, et le résidu Méthionine à la position 6 est Met-NH₂; 
His Trp Ala Trp Phe Lys
1               5
 dans lequel le résidu Tryptophane à la position 2 est D-2-méthyl-Trp, le résidu Phénylalanine à la position 5 est D-Phe, et le résidu Lysine à la position 6 est Lys-NH₂.

8. Procédé selon la revendication 4 dans lequel le peptide bioactif est 
Phe Cys Phe Trp Lys Thr Cys
1               5
 dans lequel le résidu Phénylalaline à la position 1 est D-Phe, le résidu Tryptophane à la position 4 est D-3-méthyl-Trp, et le résidu Cystéine à la position 7 est Cys-NHCH(CH₂OH)CHOHCH₃ et dans lequel les résidus Cystéine sont liés l'un à l'autre par un pont disulfure cycliquecyclique; ou 
Phe Cys Tyr Trp Lys Val Cys Trp
 1               5
 dans lequel le résidu Phénylalanine à la position 1 est D-Phe, le résidu Tryptophane à la position 4 est D-2-méthyl-Trp, et le résidu Tryptophane à la position 8 est Trp-NH₂_{,} dans lequel les résidus Cystéine sont liées l'une à l'autre par un pont disulfure cycliquecyclique.

9. Procédé selon la revendication 1 dans lequel les peptides bioactifs contiennent des résidus D-2-alkylTryptophane dans lesquels lesdits peptides sont 
Glu His Trp Ser Tyr Trp Leu Arg Pro Gly
1               5                   10
 dans lesquels le résidu acide Glutamique à la position 1 est Pyro-Glu, le résidu Tryptophane à la position 6 est D-2-méthyl-Trp, et le résidu Glycine à la position 10 est Gly-NH₂; 
Glu His Trp Ser Tyr Trp Leu Arg Pro
 1               5
 dans lequel le résidu acide Glutamique à la position 1 est Pyro-Glu, le résidu Tryptophane à la position 6 est D-2-méthyl-Trp et le résidu Proline à la position 9 est Pro-NHCH₂CH₃; 
Pro Gln Gln Trp Phe Trp Trp Met
1               5
 dans lequel le résidu Proline à la position 1 est D-Proline, les résidus Tryptophane aux positions 4 et 6 sont D-Trp, le résidu Tryptophane à la position 7 est D-2-éthyl-Trp et le résidu Méthionine à la position 8 est Met-NH₂; 
Arg Trp Phe Trp Leu Met
1               5
 dans lequel le résidu Tryptophane à la position 2 est D-Trp, le résidu Phénylalanine à la position 3 est N-méthyl-Phe, le résidu Tryptophane à la position 4 est D-2-isoPropyl-Trp, et le résidu Méthionine à la position 6 est Met-NH₂; 
Phe Cys Phe Trp Lys Thr Cys
1               5
 dans lequel le résidu Phénylalanine à la position 1 est D-Phe, le résidu Tryptophane à la position 4 est D-2-méthyl-Trp, et le résidu Cystéine à la position 7 est Cys-NHCH(CH₂OH)CHOHCH₃ et dans lequel les résidus Cystéine sont liés l'un à l'autre par un pont disulfure cyclique; 
Phe Cys Tyr Trp Lys Val Cys Trp
1               5
 dans lequel le résidu Phénylalanine à la position 1 est D-Phe, le résidu Tryptophane à la position 4 est D-2-méthyl-Trp, et le résidu Tryptophane à la position 8 est Trp-NH₂ dans lesquels les unités Cystéine sont liées l'une à l'autre par un pont disulfure cyclique;et 
His Trp Ala Trp Phe Lys
1               5
 dans lequel le résidu Tryptophane à la position 2 est D-2-méthyl-Trp, le résidu Phénylalanine à la position 5 est D-Phe, et le résidu Lysine à la position 6 est Lys-NH₂.
